# EUROPÄISCHE PATENTANMELDUNG

(11) **EP 4 729 492 A2**
(43) Veröffentlichungstag der Anmeldung: **22.04.2026**
(21) Anmeldenummer: 25206225.2
(22) Anmeldetag: 02.10.2025
(51) Int. Cl.: C03C 3/068, C03C 4/00, C03C 4/02, C03C 8/24, C03C 27/02, H01B 3/08, H01B 17/26, H01B 17/62, H01B 17/30, A61N 1/375

(54) **AUTOKLAVIERBARE ELEKTRISCHE DURCHFÜHRUNG UND GLAS FÜR EINE AUTOKLAVIERBARE ELEKTRISCHE DURCHFÜHRUNG**

(30) Priorität: 18.10.2024 DE 102024130333
(71) Anmelder: SCHOTT AG, 55122 Mainz (DE)
(72) Erfinder: HOVHANNISYAN, Martun, 55122 Mainz (DE); HOINKIS, Nina, 55122 Mainz (DE); ZETTERER, Thomas, 84028 Landshut (DE); LORENZ, Marius, 84028 Landshut (DE); HÜTSCH, Julia, 84028 Landshut (DE)
(74) Vertreter: Schott Corporate IP

(57) **Zusammenfassung**

Die Erfindung betrifft eine elektrische Durchführung (10) umfassend einen Grundkörper (20) mit zumindest einer durch den Grundkörper (20) verlaufenden Durchgangsöffnung (22), ein Isolationsmaterial (30), welches in der durch den Grundkörper (20) verlaufenden Durchgangsöffnung (22) aufgenommen ist, wobei das Isolationsmaterial (30) aus Glas besteht oder ein Glas umfasst. Ferner umfasst die elektrische Durchführung (10) zumindest einen elektrischen Leiter (40), welcher sich durch das in der Durchgangsöffnung (22) aufgenommene Isolationsmaterial (30) hindurch erstreckt, wobei der Grundkörper (20) Titan oder eine Titanlegierung umfasst. Die elektrische Durchführung (10) ist dadurch gekennzeichnet, dass das Glas ein spezielles Erdalkalimetalloxid-haltiges Lanthan-Borat-Glas ist, welches eine verbesserte hydrolytische Beständigkeit aufweist und daher den rauen Bedingungen beim vielfachen Autoklavieren standhalten kann. Die Durchführung mit dem Glas weist nach 300 Autoklavierungen einen Isolationswiderstand von mindestens 1*10⁹ Ohm auf.

## Beschreibung

Die Erfindung betrifft eine autoklavierbare elektrische Durchführung mit einem metallumfassenden Grundkörper, in welchem ein Glas umfassendes Isolationsmaterial mit einem sich hindurch erstreckenden elektrischen Leiter aufgenommen ist. Ferner betrifft die Erfindung ein Glas für eine solche autoklavierbare elektrische Durchführung.

Elektrische Durchführungen mit einem äußeren metallischen Grundkörper und einer als Isolator dienenden inneren Glaskomponente (Glas-Metall-Durchführungen), durch welche sich ein oder mehrere Leiter erstrecken, kommen bei zahlreichen Anwendungen, insbesondere für hermetische Wandungsteile, etwa von Gehäuseelementen, zum Einsatz. Entsprechende Bauteile finden beispielsweise Verwendung im Bereich der Medizintechnik, z.B. in elektrischen Steckverbinder und hermetischen Gehäuseteilen/Dichtungen in medizinischen Geräten, wie in Endoskopen und in chirurgischen Instrumenten, die zum Untersuchen, Operieren und Behandeln verwendet werden und nur kurzzeitig mit dem Körper Kontakt haben, aber dafür öfter nach entsprechender Sterilisation wiederverwendet werden können. Ein Einsatzgebiet ist z. B. in der Roboterchirurgie. Weitere Einsatzgebiete sind implantierbare medizinische Geräte (IMD), Leichtbauanwendungen (z.B. im Bereich Luft- und Raumfahrt) sowie viele andere Bereiche. Je nach Einsatzgebiet können unterschiedliche Anforderungsprofile zu berücksichtigen sein, wobei jedoch auch Gemeinsamkeiten im Hinblick auf die Optimierung und Weiterentwicklung bekannter Lösungen bestehen.

Im Hinblick auf die Verwendung von Durchführungen in Geräten der Medizintechnik bestehen besondere Anforderungen. In der Regel werden in diesem Bereich die Durchführungen in einem Bauteil aus Titan oder einer Titan-Legierung vorgesehen. Für Einglasungen in Titan oder Titanlegierungen bestehen besondere Herausforderungen, da das Material Titan stark reaktiv ist. Bei hoch silikathaltigen Gläsern besteht das Problem, dass das Titan bei den üblichen Einglasungstemperaturen (engl. "sealing temperatures") von ca. 700-900°C, die für die Herstellung der Glas-Metall-Durchführungen nötig sind, mit SiO₂ zu Titansilizid reagiert, und diese Reaktion von einer Blasenbildung am Interface Glas-Metall begleitet wird. Außerdem ist zu berücksichtigen, dass reines Titan eine Phasenübergangstemperatur von 880°C (Übergang der Tieftemperaturmodifikation alpha-Titan in die Hochtemperaturmodifikation beta-Titan) aufweist. Für Titan-Legierungen z.B. Titan Grade 5 (TiAl6V4) ist diese Phasenübergangstemperatur höher, aber die Reaktionsmechanismen am Interface zum Glas sind die gleichen. Daher müssen die Glaszusammensetzungen im Hinblick auf niedrige Einglasungstemperaturen optimiert werden, um die Einglasung unterhalb der für die Versiegelungsfähigkeit nachteilige Übergangsreaktion durchführen zu können.

Außerdem ist es in der Regel wünschenswert und ein Aspekt der Aufgabe der Erfindung, dass in der Medizintechnik einsetzbare Geräte bzw. Bauteile mit Glas-Metall-Durchführungen autoklavierbar sind, d.h. bei hohem Dampfdruck und Temperaturen > 100°C sterilisierbar sind, um die Geräte mehrfach für Diagnosen oder Behandlungen am oder im menschlichen oder tierischen Körper oder für die Untersuchung z.B. von biologischem Material in Laboren usw. einsetzen zu können. Das Autoklavieren über viele Zyklen bzw. Durchläufe hinweg, d.h. das wiederholte Autoklavieren, stellt eine hohe Belastung für die verwendeten Materialien und insbesondere für das Glas in den Durchführungen dar. Bekannte Gläser, die in Durchführungen in medizinischen Geräten eingesetzt werden, weisen eine relativ geringe Autoklavierungsstabilität auf, d.h., aufgrund einer geringen chemischen Stabilität der Gläser nimmt der Isolationswiderstand, den das Glas in der Durchführung bietet, nach relativ wenigen Autoklavierungen derart ab, dass das Gerät nicht mehr einsetzbar ist. An vielfach genutzte Medizingeräte, die nicht dauerhaft im Körper verbleiben und nach dem Gebrauch viele Male sterilisiert werden müssen, werden somit in dieser Hinsicht höhere Anforderungen gestellt als an implantierbare medizinische Geräte (IMD, implantable medical devices), z.B. Herzschrittmacher, Defibrillatoren, Pumpen etc.

Außerdem ist es in der Regel wünschenswert, dass die eingesetzten Komponenten nicht toxisch wirken, wenn sie zumindest zeitweise oder auch dauerhaft (bei Implantaten) mit Körperflüssigkeiten in Berührungen kommen können. Dies betrifft neben dem Grundkörper und den elektrischen Leitern insbesondere die Gläser (aufgrund von Auslaugungseffekten), weshalb es hier insbesondere vorgesehen sein kann, dass Auslaugungstests vorab durchgeführt werden, bevor z.B. weiterführende Qualifikationstests für z.B. medizinische Geräte und Implantate durchgeführt werden. Moderne Durchführungen für medizinische Anwendungen enthalten meist Bor-Aluminiumsilikatgläser. Sie sind aufgrund der erforderlichen hohen Stabilität gegenüber wässrigen Lösungen häufig alkalifrei und weisen einen hohen Gehalt an B₂O₃, Al₂O₃ und Erdalkalimetalloxiden (CaO, MgO, SrO) auf. In bekannten Auslaugungstest wird in der Regel die Auslaugung in Wasser bestimmt, indem z.B. über den Gewichtsverlust von polierten Glasproben nach einer Verweildauer von zwei Wochen in deionisiertem Wasser bei 70°C die Lösungsrate bestimmt wird (siehe unten). Eine geringere Auflösungsrate einer Probe soll dabei mit einer erhöhten Widerstandsfähigkeit der Glaszusammensetzung gegenüber chemischen Angriffen durch Feuchtigkeit, Wasser oder wasserhaltige Körperflüssigkeiten (d. h. einer erhöhten Wasserbeständigkeit) korrelieren.

Bei autoklavierbaren Bauteilen mit elektrischen Durchführungen werden jedoch wesentlich höhere Anforderungen an die chemische Beständigkeit der Gläser gestellt. Die Gläser werden durch die Dampfdrucksterilisation bei hohen Temperaturen (in der Regel zwischen 110 und 140°C) in hohem Maße beansprucht. Wenn die hydrolytische Beständigkeit gering ist, führt das kontinuierliche Herauslösen von Glaskomponenten während der vielfachen Autoklavierungsdurchläufe dazu, dass der Isolationswiderstand der Durchführung unter einen kritischen Wert sinkt, so dass das Gerät nicht mehr sicher genutzt werden kann.

Eine weitere Anforderung an ein Glas, das für Durchführungen in Geräten der Medizintechnik geeignet sein soll, ist der thermische Ausdehnungskoeffizient, der auf die zum Einsatz kommenden Metallbauteile und auf die gewünschte Art der Druckverhältnisse in der Durchführung abgestimmt sein sollte.

US2009/0229858A1, US2020/0261732A1 und US2021/0290964A1 offenbaren eine implantierbare medizinische Vorrichtung mit einer Glas-Metall-Durchführung in einem Titan-Bauteil, wobei das Glas ein erdalkalimetallhaltiges Bor-Aluminiumsilikatglas ist und Füllstoffe zur Anpassung des thermischen Ausdehnungskoeffizienten (CTE) aufweisen kann. Es werden somit Komposit-Materialien offenbart. Die CTE-Anpassung ist Schwerpunkt der US2020/0261732A1. Die US2021/0290964A1 lehrt, wie CTE und Elastizitätsmodul des Komposits durch unterschiedliche Gehalte an kristallinem Füllstoff, explizit durch zugegebene Al₂O₃ Partikel, variiert werden. Die Beständigkeit der dort offenbarten Gläser gegenüber Dampfdrucksterilisation bei Temperaturen ist schlecht. Da jedoch implantierbare medizinische Vorrichtungen bis zum dauerhaften Verbleib im Körper nur wenigen Sterilisationsdurchläufen Stand halten müssen, sind solche Gläser trotz ihrer schlechten hydrolytischen Beständigkeit für diese Anwendungen ausreichend. Für Bauteile mit Durchführungen, die mehrere 100 Male autoklaviert werden sollen, sind solche Gläser jedoch nicht geeignet. D.h. die Autoklavierungsstabilität der Durchführung mit für medizinische Implantate geeigneten Gläsern genügt nicht den Anforderungen, die an vielfach genutzte Medizingeräte gestellt werden, die nicht im Körper verbleiben und nach dem Gebrauch viele Male sterilisiert werden müssen.

US5648302B offenbart eine Glaszusammensetzung für eine hermetische Glas-zu-Metall-Durchführung mit Titan und Titanlegierungen für implantierte medizinische Geräte mit Beständigkeit gegenüber wässrigen Lösungen (bestimmt in Auslaugungstests mit polierten Glasproben in deionisiertem Wasser bei 70°C). Bei dem Glas handelt es sich um ein Barium-Lanthan-Boratglas. Aufgrund des hohen BaO-Gehalts des Glases werden solche Gläser bei hohen Temperaturen (> 100°C) durch Wasser stark angegriffen, d.h. die hydrolytische Beständigkeit des Glases bei hohen Temperaturen, wie sie beim Autoklavieren herrschen, ist relativ gering, d.h. die Glas-Metall-Durchführung kann nur einige Male (z.B. < 100) autoklaviert werden, bevor der Isolationswiderstand unzureichend wird, was für implantierbare Anwendungen ausreichend ist, aber nicht für vielfach wiederverwendbare Geräte z.B. in der Medizintechnik.

US5693580B offenbart eine Glaszusammensetzung für eine hermetische Glas-zu-Metall-Durchführung mit Titan und Titanlegierungen für implantierte medizinische Geräte mit hoher Beständigkeit gegenüber wässrigen Lösungen (bestimmt in Auslaugungstests mit polierten Glasproben in deionisiertem Wasser bei 70°C). Bei dem Glas handelt es sich um ein Calcium-Lanthan-Boratglas. Die dort offenbarten Gläser enthalten kein SiO₂, um die Bildung von Titansilizid zu vermeiden. Auch diese Gläser zeigen eine geringe hydrolytische Beständigkeit bei Temperaturen > 100°C, d.h. eine Autoklavierungsstabilität über viele Durchläufe hinweg ist nicht gegeben.

In der US10544058B1 werden spezielle Komposit-Materialien offenbart, die ein Alkali-Aluminosilikatglas und Füllstoffe umfassen. Aufgrund des Alkaligehalts und des Füllstoffgehalts, der zu einer gewissen Porosität führen kann, sind solche Gläser weniger für Anwendungen, die eine hohe Autoklavierungsstabilität der Durchführungen erfordern, geeignet.

Außerdem sind implantierbare medizinische Geräte bekannt, z.B. aus US2015/0088226 A ein Cochlea-Implantat, das ein abgedichtetes Gehäuse mit Elektronik und Durchführung mit Keramik als Isolationsmaterial umfasst.

In der WO2023/016964A wird eine Durchführung beschrieben, die einen Grundkörper aus Titan oder einer Titanlegierung mit Durchgangsöffnung, ein Glas als Isolationsmaterial und mindestens einen elektrischen Leiter umfasst, wobei das Glas mit dem Metallteilen einen besonderen Kontaktwinkel ausbildet. Das dort offenbarte Alkali-Aluminosilikatglas weist aufgrund des Alkaligehalts eine relativ geringe Beständigkeit gegenüber vielfachem Autoklavieren auf.

Allgemein ist es z.B. wünschenswert und eine Aufgabe der vorliegenden Erfindung, die Beständigkeit eingangs genannter Durchführungen hinsichtlich chemischer und/oder physikalischer Einflüsse zu erhöhen und die Dichtigkeit des Isolationsmaterials zu dem umschließenden Grundkörper und/oder zu dem sich durch das Isolationsmaterial hindurch erstreckenden elektrischen Leiter zu verbessern. Insbesondere ist es die Aufgabe der vorliegenden Erfindung, eine Durchführung bereitzustellen, die vielfach autoklavierbar ist und dennoch den benötigten Isolationswiderstand behält. Ferner ist es Aufgabe, ein für eine vielfache Autoklavierung geeignetes Glas bereitzustellen, das für die Verbindung mit einem Grundkörper aus Titan oder einer Titanlegierung geeignet ist.

### Offenbarung der Erfindung

Zur Lösung der Aufgabe wird mit der vorliegenden Erfindung eine elektrische Durchführung bereitgestellt, welche einen Grundkörper mit zumindest einer durch den Grundkörper verlaufenden Durchgangsöffnung umfasst, ferner ein Isolationsmaterial, welches in der durch den Grundkörper verlaufenden Durchgangsöffnung aufgenommen ist, sowie zumindest einen elektrischen Leiter welcher sich durch das in der Durchgangsöffnung aufgenommene Isolationsmaterial hindurch erstreckt, wobei der Grundkörper Titan oder eine Titanlegierung umfasst und das Isolationsmaterial Glas umfasst oder aus Glas besteht.

Das Glas ist ein Lanthan-Borat-Glas, das folgende Komponenten (in Mol-% auf Oxidbasis) umfasst

| | | | |
|---|---|---|---|
| B₂O₃ | 22,0 | - | 37,0 |
| La₂O₃ | 1,0 | - | 12,0 |
| SiO₂ | 10,5 | - | 23,0 |
| RO (MgO+CaO+SrO) | 29,0 | - | 45,0 |
| Nb₂O₅+ZrO₂+TiO₂+Ta₂O₅ | 0,1 | - | 6,0 |
| SiO₂/(Nb₂O₅+ZrO₂+TiO₂+Ta₂O₅) | > 3,00 | | |

wobei das Glas bei Bestimmung der hydrolytischen Beständigkeit gemäß DIN ISO 720:2021-12 einen Na₂O Äquivalenzwert von < 1250 µg/g aufweist, so dass die Durchführung mit einem solchen Glas Langzeit-autoklavierungsbeständig ist,
wobei bevorzugt ein Isolationswiderstand der Durchführung nach 300 Autoklavierungen mindestens 1*10⁹ Ohm beträgt.

Die Erfinder haben herausgefunden, dass sich überraschenderweise die hydrolytische Beständigkeit eines Lanthan-Borat-Glases gegenüber den rauen Bedingungen, die beim Autoklavieren herrschen, deutlich verbessern lässt, wenn das Glas einen für eine Glas-Metall-Durchführung mit Titan oder einer Titanlegierung relativ hohen Anteil an SiO₂ und einen bestimmten Anteil an Nb₂O₅ und/oder ZrO₂ und/oder TiO₂ und/oder Ta₂O₅ aufweist. Die genannten Komponenten wirken einzeln oder in Kombination - d.h. mindestens zwei dieser Komponenten - als Beständigkeitsverstärker für die hydrolytische Beständigkeit. Mit anderen Worten, der Beständigkeitsverstärker weist mindestens eine Komponente auf, ausgewählt aus der Gruppe umfassend Nb₂O₅, ZrO₂, TiO₂, Ta₂O₅. Dabei sind diese Komponenten erfindungsgemäß Bestandteil des Glases, d.h. es sind keine Füllstoffe, die nachträglich zugesetzt sind. Im Unterschied zu einem Glas bzw. Isolationsmaterial mit Füllstoffen, weist das erfindungsgemäße Glas bzw. das Isolationsmaterial in der Durchführung daher nur wenige Poren auf, was vorteilhaft für die hermetische Dichtheit ist. Eine Durchführung mit einem solchen Glas ist vorteilhaft Langzeit-autoklavierungsbeständig, so dass sie in einem Gerät, Bauteil etc. in der Medizintechnik, das vielfach autoklavierbar sein muss, eingesetzt werden kann. Vorteilhaft kann das Glas bzw. das Isolationsmaterial porenfrei sein.

Die hydrolytische Beständigkeit des Glases wird gemäß DIN ISO 720:2021-12 an Glasgrieß bei 121°C bestimmt, d.h. bei ähnlich hohen Temperaturen wie beim Autoklavieren. Dabei wird Glasgrieß mit einer definierten Körnung und Oberfläche im Autoklaven bei 121°C für 30 Min in destilliertem Wasser gehalten. Die Beständigkeit wird gemessen und ausgedrückt durch das Volumen an Säure, das für die Titration der aus der Masseneinheit Glas extrahierten Lauge erforderlich ist, und kann auch durch die diesem Volumen an Säure entsprechende Menge an Natriumoxid ausgedrückt werden. Im Rahmen der Erfindung wird auf den Natriumoxid-Äquivalenzwert ausgedrückt als Masse an Natriumoxid pro Gramm Glaskörner abgestellt.

Die erfindungsgemäßen Gläser weisen einen Na₂O Äquivalenzwert von < 1250 µg/g auf. Vorteilhafte Ausführungen weisen einen Na₂O Äquivalenzwert von < 1100 µg/g oder < 1000 µm/g auf. Besonders vorteilhafte Varianten weisen einen Na₂O Äquivalenzwert von < 930 µg/g auf und sind damit gemäß DIN ISO 720:2021-12 in die Klasse HGA 3 einzustufen. - Vorteilhaft weist das Glas einen Na₂O Äquivalenzwert auf, so dass die Durchführung mit einem solchen Glas Langzeit-autoklavierungsbeständig ist.

"Langzeit-autoklavierungsbeständig" im Sinne der Erfindung bedeutet, dass die Durchführung mindestens 300-mal bei 135,5°C und einem Druck von 2,16 bar für eine Dauer von 20 min autoklaviert werden kann unter Beibehaltung eines ausreichenden Isolationswiderstandes. Damit erfüllen die Durchführungen die Anforderungen, die an vielfach genutzte Gesundheitsgeräte, Medizinprodukte und Laborausrüstung gestellt werden. Die Beständigkeit gegenüber vielfachem Autoklavieren wird bestimmt, wie weiter unten im Zusammenhang mit den Ausführungsbeispielen beschrieben. Zur Vermeidung von Wiederholungen wird darauf verwiesen.

Vorteilhaft beträgt der Isolationswiderstand der Durchführung nach 300 oder nach mindestens 300 Autoklavierungen immer noch mindestens 1*10⁹ Ohm. Wenn der Isolationswiderstand unterhalb dieser Grenze liegt, sind Leiter (auch Pin genannt) und Außenleiter in einer Durchführung nicht mehr ausreichend isoliert, so dass es zu Leckströmen bis hin zum Kurzschluss kommen kann. Mit dem genannten Mindest-Isolationswiderstand lässt sich ein Gerät, das die erfindungsgemäße Langzeit-autoklavierungsbeständige Durchführung enthält, sicher betreiben. Durchführungen, die mindestens 300 Autoklavierungsdurchläufe bei den oben genannten Bedingungen Stand halten, können auch vielfaches Sterilisieren bei höheren Temperaturen und/oder höheren Drücken und/oder über längere Zeiträume als oben beschrieben aushalten.

Vorteilhaft beträgt der Isolationswiderstand der Durchführung nach mindestens 600, bevorzugt nach mindestens 900, bevorzugt nach mindestens1200 Autoklavierungen immer noch mindestens 1x10⁹ Ohm, wobei die Sterilisation jeweils unter den oben genannten Bedingungen durchgeführt wurde (d.h. 135,5°C; 2,16 bar; 20 min).

Der Isolationswiderstand, der ein Maß für die Autoklavierungsstabilität ist, wird nach MIL-STD-883, Meth. 1003 an der Durchführung mit eingeglastem Leiter bestimmt, wobei die Elektroden am Grundkörper und am Leiter platziert sind. Die Messung wird jeweils an einer Stelle bei Raumtemperatur (20°C) und einer Feuchtigkeit unter typischen Laborbedingungen (50% +/- 10% relative Luftfeuchtigkeit) mit einer Spannung von 100 V, Messdauer von 5 s durchgeführt. Für die Bestimmung des Isolationswiderstandes wird im Rahmen der Erfindung immer eine elektrische Durchführung mit folgenden Abmessungen eingesetzt: Durchmesser der das Isolationsmaterial mit Leiter aufnehmenden Durchgangsöffnung im Grundkörper 1,8 mm, Durchmesser des Leiters 0,5 mm und Einglasungslänge 3,0 mm. - Selbstverständlich können in der Anwendung die Dimensionen anders sein.

In Bezug auf Hermetizität kann vorteilhaft vorgesehen sein, dass die Durchführung nach 300 oder nach mindestens 300 Autoklavierungsdurchläufen eine Hermetizität aufweist, welche gekennzeichnet ist durch eine Helium-Leckrate von weniger als 1·10⁻⁸ mbar·l/s, vorzugsweise von weniger als 1·10⁻⁹ mbar·l/s, besonders bevorzugt von weniger als 1·10⁻¹⁰ mbar·l/s, bestimmt bei einem Druckunterschied von 1 bar. Die Hermetizität von Durchführungen kann beispielsweise durch einen Helium-Lecktest bestimmt werden, beispielsweise nach MIL-STD-883, Meth. 1014 A4. Eine Helium-Leckrate von weniger als 1·10⁻⁸ mbar·l/s wird vorteilhafterweise auch noch nach mindestens 600, vorteilhaft mindestens 900, vorteilhaft mindestens 1200 Autoklavierungen erreicht. D.h., dass das in der Durchgangsöffnung des Grundkörpers aufgenommene Isolationsmaterial mit dem Grundkörper und/oder mit dem zumindest einen elektrischen Leiter in innigem Kontakt steht. Vorteilhafterweise schmilzt das Glas bei der Herstellung der Durchführung und glast an den Metallkomponenten an, so dass eine dauerhafte, hermetisch dichte Glas-Metall-Verbindung vorliegt.

### Glaszusammensetzung

Die im Folgenden beschriebenen Zusammensetzungsmerkmale und vorteilhaften Ausführungen tragen zu den erfindungsgemäßen und vorteilhaften Eigenschaften des Glases bei. Die Beschreibung gilt sowohl für das Glas als auch für eine Durchführung mit einem solchen Glas. Es wurde gefunden, dass bei einem Erdalkalimetalloxid-haltigen Lanthan-Borat-Glas insbesondere durch einen definierten Gehalt einer oder mehrerer bestimmter Komponenten, die als Glaskomponente(n) als Beständigkeitsverstärker wirken, und durch einen gewissen Anteil an SiO₂ die chemische Beständigkeit des Glases derart erhöht werden kann, dass es den rauen Bedingungen beim vielfachen Autoklavieren standhalten kann. Außerdem erfüllt es weitere gewünschte vorteilhafte Eigenschaften, z.B. im Hinblick auf hermetische Dichtheit der Durchführung, Einglasungstemperatur für die Herstellung der Durchführung, thermische Ausdehnung etc.

Die Einglasungstemperatur für die Herstellung der Glas-Metall-Durchführung beträgt vorteilhaft ≤ 950°C, vorteilhaft ≤ 930°C, vorteilhaft ≤ 900°C, vorteilhaft ≤ 880°C. Die Einglasungstemperatur ist dabei die Temperatur, bei der erfolgreich hermetisch dichte Durchführungen hergestellt werden können. Ein Maß für die Einglasungstemperatur ist die Halbkugeltemperatur (englisch "half spherical temperature"). Erfahrungsgemäß deckt sich die Einglasungstemperatur in etwa mit der Halbkugeltemperatur bzw. liegt einige Grad darüber, beispielsweise mindestens 5°C oder mindestens 25°C oder mindestens 50°C oder mindestens 70°C.

Das erfindungsgemäße Glas enthält B₂O₃. Vorteilhaft ist diese Komponente mit einem Anteil von 22,0 bis 37,0 Mol% enthalten. B₂O₃ ist eine Glaskomponente, die für die Glasbildung und die hydrolytische Beständigkeit wichtig ist. Außerdem kann B₂O₃ mit Titan eine Ti-B-Schicht bilden, die zu chemisch und mechanisch stabileren Verbindungen zwischen titanumfassenden Komponenten und dem Glas führt. Daher ist diese Komponente vorteilhaft mit mindestens 22,0 Mol% oder mindestens 22,5 Mol% im Glas enthalten ist. Eine Obergrenze von 37,0 Mol% sollte nicht überschritten werden, weil ansonsten Gehalte an anderen Glaskomponenten, insbesondere Erdalkalimetalloxiden und/oder Al₂O₃, reduziert werden müssten, was zu einer Verschlechterung angestrebter Glaseigenschaften führen würde, z.B. zu einer Erniedrigung des thermischen Ausdehnungskoeffizienten, Anstieg der Einglasungstemperatur etc.. In manchen vorteilhaften Varianten kann das Glas einen Gehalt an B₂O₃ von mindestens 25,0 Mol%, mindestens 27,0 Mol%, mindestens 28,0 Mol% haben. Der Gehalt kann in einer vorteilhaften Ausführung höchstens 37,0 Mol% oder höchstens 35,0 Mol% betragen. Für manche vorteilhaften Varianten kann auch 31,0 Mol% oder weniger als 31,0 Mol% eine geeignete Obergrenze sein.

Das erfindungsgemäße Glas enthält La₂O₃ mit einem Anteil von 1,0 bis 12,0 Mol%. La₂O₃ ist eine Glaskomponente, die den Glasfluss beim Schmelzen verbessert und die Einglasungstemperatur, erniedrigt, so dass die Einglasung des Leiters in den Grundkörpers bei niedrigeren Temperaturen erfolgen kann. Außerdem kann diese Komponente den thermischen Ausdehnungskoeffizienten des Glases anheben. Aus diesen Gründen ist diese Komponente vorteilhaft mit mindestens 1,0 Mol% im Glas enthalten. Eine Obergrenze von 12,0 Mol% sollte nicht überschritten werden, weil ansonsten die Einglasungstemperatur zu hoch ist. In einer vorteilhaften Ausführungsform hat das Glas einen Gehalt an La₂O₃ von mindestens 1,5 Mol% oder mindestens 2,0 Mol%. Der Gehalt kann in einer vorteilhaften Ausführung optional höchstens 11,0 Mol% oder höchstens 10,0 Mol% oder höchstens 9,0 Mol% oder höchstens 8,0 Mol% oder weniger als 8,0 Mol% betragen.

Das erfindungsgemäße Glas enthält SiO₂ mit einem Gehalt von 10,5 bis 23,0 Mol%. Ein Mindestgehalt von 10,5 Mol% sollte nicht unterschritten werden, da ansonsten die hydrolytische Beständigkeit des Glases und damit der Autoklavierungsbeständigkeit der Durchführung nicht erreicht wird. Eine Obergrenze von 23,0 Mol% sollte nicht überschritten werden, da mit zunehmendem SiO₂-Gehalt die für die Einglasung des Leiters in den Grundkörper nötigen Temperaturen ansteigen, so dass die Gefahr besteht, dass im Titan oder in der Titanlegierung des Grundkörpers - und ggf. des Leiters - der unerwünschte Phasenübergang (alpha Titan - beta Titan) mit dem damit verbundenen negativen Eigenschaftsänderungen eintritt oder Titan mit SiO₂ zu Titansilizid reagiert. Es kann zu Volumenänderungen und/oder zur Blasenbildung im Glas an den Grenzflächen mit dem Metall kommen. Außerdem erniedrigt ein zu hoher SiO₂ Gehalt den thermischen Ausdehnungskoeffizienten. In einer vorteilhaften Ausführungsform hat das Glas einen Gehalt an SiO₂ von mindestens 11,0 Mol% oder mindestens 11,5 Mol% oder mindestens 12,0 Mol%. In vorteilhaften Varianten kann der Mindestgehalt an SiO₂ 15,0 Mol% oder mehr als 15,0 Mol% betragen. Für manche vorteilhafte Varianten kann 15,5 Mol% oder 16,0 Mol% eine Untergrenze sein. Der SiO₂-Gehalt kann in einer vorteilhaften Ausführung optional höchstens 22,5 Mol%, vorteilhaft höchstens 22,0 Mol%, vorteilhaft höchstens 21,0 Mol%, für manche Varianten höchstens 20,0 Mol% betragen.

Das erfindungsgemäße Glas enthält einen Beständigkeitsverstärker mit einem Gehalt von 0,1 bis 6,0 Mol%, wobei der Beständigkeitsverstärker mindestens eine Komponente ist, ausgewählt aus der Gruppe umfassend Nb₂O₅, ZrO₂, TiO₂, Ta₂O₅. Die Summe der Komponenten Nb₂O₅ + ZrO₂+ TiO₂ + Ta₂O₅ sollte mindestens 0,1 Mol% betragen, da ansonsten die hydrolytische Beständigkeit nicht ausreichend verbessert ist. Eine vorteilhafte Untergrenze kann auch 0,2 Mol% oder 0,3 Mol% oder für manche Varianten 0,4 Mol% sein. Eine Obergrenze von 6,0 Mol% sollte nicht überschritten werden, da das Risiko steigt, dass eine Entglasung, d.h. Kristallbildung, stattfindet. Außerdem steigt mit zunehmendem Gehalt die Einglasungstemperatur an mit den oben für die Komponente SiO₂ beschriebenen Nachteilen. Eine vorteilhafte Obergrenze für die Summe kann auch 5,5 Mol% oder 5,0 Mol% oder 4,5 Mol% sein. Der Beständigkeitsverstärker ist Bestandteil des erschmolzenen Glases, d.h. eine Glaskomponente.

Als Beständigkeitsverstärker kann das Glas vorteilhaft Nb₂O₅ enthalten mit einem Gehalt von 0,0 bis 6,0 Mol%. Wenn das Glas Nb₂O₅ enthalten soll, kann die Komponente vorteilhaft mit mindestens 0,05 Mol% oder mindestens 0,1 Mol% oder mindestens 0,2 Mol% enthalten sein. In einer vorteilhaften Ausführungsform ist der Gehalt an Nb₂O₅ auf maximal 6,0 Mol%, vorteilhaft maximal 5,5 Mol%, vorteilhaft maximal 5,0 Mol%, vorteilhaft maximal 4,5 Mol% oder maximal 4,0 Mol% begrenzt. In einer vorteilhaften Ausführungsform ist das Glas frei von Nb₂O₅. Es kann vorteilhaft sein, den Gehalt an Nb₂O₅ zu reduzieren oder ganz auf Nb₂O₅ zu verzichten, um eine Kristallbildung im Glas und/oder ein Ansteigen der Einglasungstemperatur zu verhindern.

Als Beständigkeitsverstärker kann das Glas vorteilhaft ZrO₂ enthalten mit einem Gehalt von 0,0 bis 6,0 Mol%. Wenn das Glas ZrO₂ enthalten soll, kann die Komponente vorteilhaft mit mindestens 0,05 Mol% oder mindestens 0,1 Mol% oder mindestens 0,2 Mol% enthalten sein. In einer vorteilhaften Ausführungsform ist der Gehalt an ZrO₂ auf maximal 6,0 Mol%, vorteilhaft maximal 5,5 Mol%, vorteilhaft maximal 5,0 Mol%, vorteilhaft maximal 4,5 Mol%, vorteilhaft maximal 4,0 Mol% oder maximal 3,5 Mol% begrenzt. In einer vorteilhaften Ausführungsform ist das Glas frei von ZrO₂. Es kann vorteilhaft sein, den Gehalt an ZrO₂ zu reduzieren oder ganz auf ZrO₂ zu verzichten, um eine Kristallbildung im Glas und/oder ein Ansteigen der Einglasungstemperatur zu verhindern.

Als Beständigkeitsverstärker kann das Glas vorteilhaft TiO₂ enthalten mit einem Gehalt von 0,0 bis 6,0 Mol%. Wenn das Glas TiO₂ enthalten soll, kann die Komponente vorteilhaft mit mindestens 0,05 Mol% oder 0,1 Mol% oder mindestens 0,2 Mol% enthalten sein. In einer vorteilhaften Ausführungsform ist der Gehalt an TiO₂ auf maximal 6,0 Mol%, vorteilhaft maximal 5,5 Mol%, vorteilhaft maximal 5,0 Mol%, vorteilhaft maximal 4,5 Mol% oder maximal 4,0 Mol% begrenzt. In einer vorteilhaften Ausführungsform ist das Glas frei von TiO₂. Es kann vorteilhaft sein, den Gehalt an TiO₂ zu reduzieren oder ganz auf TiO₂ zu verzichten, um eine Kristallbildung im Glas zu verhindern.

Als Beständigkeitsverstärker kann das Glas vorteilhaft Ta₂O₅ enthalten mit einem Gehalt von 0,0 bis 6 Mol%. Wenn das Glas Ta₂O₅ enthalten soll, kann die Komponente vorteilhaft mit mindestens 0,05 Mol% oder mindestens 0,1 Mol% oder mindestens 0,2 Mol% enthalten sein. In einer vorteilhaften Ausführungsform ist der Gehalt an Ta₂O₅ auf maximal 6,0 Mol%, vorteilhaft maximal 5,5 Mol%, vorteilhaft maximal 5,0 Mol%, vorteilhaft maximal 4,5 Mol%, vorteilhaft maximal 4,0 Mol% oder maximal 3,5 Mol% begrenzt. In einer vorteilhaften Ausführungsform ist das Glas frei von Ta₂O₅. Es kann vorteilhaft sein, den Gehalt an Ta₂O₅ zu reduzieren oder ganz auf Ta₂O₅ zu verzichten, um die Einglasungstemperatur gering zu halten und um eine Kristallbildung im Glas zu verhindern.

Das erfindungsgemäße Glas enthält RO mit einem Gehalt von 29,0 bis 45,0 Mol%, wobei RO für die Gesamtsumme der Erdalkalimetalloxide MgO+CaO+SrO steht. Mit Erdalkalimetalloxiden lassen sich die Temperatur, bei der die Einglasung des Leiters in den Grundkörper erfolgen kann, erniedrigen und der thermische Ausdehnungskoeffizient anheben, weshalb mindestens 29,0 Mol% an RO enthalten sind. Eine Obergrenze von 45,0 Mol% sollte nicht überschritten werden, da sich andernfalls die chemische Beständigkeit des Glases verschlechtert und die Gefahr einer Kristallbildung im Glas (Entglasung) besteht. In einer vorteilhaften Ausführungsform enthält das Glas mindestens 30,0 Mol% oder mindestens 31,0 Mol% an RO. Vorteilhaft kann der Gehalt auf maximal 42,0 Mol% oder maximal 40,0 Mol% oder für manche Varianten maximal 39,5 Mol% oder maximal 39,0 Mol% oder maximal 38,0 Mol% oder maximal 37,0 Mol% begrenzt sein.

Das Glas kann in einer vorteilhaften Ausführung MgO mit einem Gehalt von 0,0 bis 20,0 Mol% enthalten, d.h. der Gehalt an MgO beträgt maximal 20,0 Mol%. In einer vorteilhaften Ausführungsform ist der Gehalt an MgO auf maximal 18,0 Mol%, maximal 17,0 Mol%, maximal 16,0 Mol% begrenzt. Wenn das Glas MgO enthalten soll, kann in manchen Varianten vorteilhaft mindestens 0,1 Mol% oder mindestens 0,2 Mol% MgO enthalten sein. In vorteilhaften Varianten sind mindestens 4,0 Mol% oder mindestens 5,0 Mol% oder mindestens 6,0 Mol% MgO enthalten. Manche vorteilhaften Varianten können auch mindestens 8,0 Mol% oder mindestens 9,0 Mol% an MgO enthalten. Es kann vorteilhaft sein, einen gewissen Gehalt an MgO neben CaO und/oder SrO im Glas vorzusehen, um durch die Kombination eine Kristallbildung im Glas zu verhindern. Ein vorteilhafter Bereich für MgO kann 6,0 bis 18,0 Mol% sein. In einer alternativen vorteilhaften Ausführungsform kann das Glas frei von MgO sein.

Das Glas kann in einer vorteilhaften Ausführung CaO mit einem Gehalt von 0,0 bis 20,0 Mol% enthalten, d.h. der Gehalt an CaO beträgt maximal 20,0 Mol%. In einer vorteilhaften Ausführungsform ist der Gehalt an CaO auf maximal 19,0 Mol% oder maximal 18,0 Mol% oder für manche Varianten maximal 17,0 Mol% begrenzt. Wenn das Glas CaO enthalten soll, kann in manchen Varianten vorteilhaft mindestens 0,1 Mol% oder mindestens 0,2 Mol% CaO enthalten sein. In vorteilhaften Varianten sind mindestens 5,0 Mol% oder mindestens 7,0 Mol% oder mindestens 10,0 Mol% CaO enthalten. Es kann vorteilhaft sein, einen gewissen Gehalt an CaO neben MgO und/oder SrO im Glas vorzusehen, um durch die Kombination eine Kristallbildung im Glas zu verhindern. Ein vorteilhafter Bereich für CaO kann 10,0 bis 20,0 Mol% sein. In einer alternativen vorteilhaften Ausführungsform kann das Glas frei von CaO sein

Das Glas kann in einer vorteilhaften Ausführung SrO mit einem Gehalt von 0,0 bis 10,0 Mol% enthalten, d.h. der Gehalt an SrO beträgt maximal 10,0 Mol%. In einer vorteilhaften Ausführungsform ist der Gehalt an SrO auf maximal 9,0 Mol% oder maximal 8,0 Mol% begrenzt. Wenn das Glas SrO enthalten soll, kann in manchen Varianten vorteilhaft mindestens 0,1 Mol% oder mindestens 0,2 Mol% SrO enthalten sein. In vorteilhaften Varianten sind mindestens 2,0 Mol% oder mindestens 3,0 Mol% oder mindestens 4,0 Mol% SrO enthalten. Es kann vorteilhaft sein, einen gewissen Gehalt an SrO neben MgO und/oder CaO im Glas vorzusehen, um durch die Kombination eine Kristallbildung im Glas zu verhindern. Ein vorteilhafter Bereich für SrO kann 3,0 bis 8,0 Mol% sein. In einer alternativen vorteilhaften Ausführungsform kann das Glas frei von SrO sein.

Um die Entglasungsgefahr zu verringern, enthält das Glas vorteilhaft mindestens zwei Erdalkalimetalloxide in beliebiger Kombination, ausgewählt aus der Gruppe bestehend aus MgO, CaO und SrO. Besonders bevorzugt enthält das Glas MgO und CaO und SrO.

Das Glas kann vorteilhaft 0,0 bis maximal 1,5 Mol% BaO enthalten. Diese Grenze sollte nicht überschritten werden, da diese Komponente die hydrolytische Beständigkeit des Glases verringern kann, so dass die gewünschte Langzeit-Autoklavierungsstabilität der Durchführung nicht mehr gegeben ist. Vorteilhaft enthält das Glas maximal 1,0 Mol%, vorzugsweise maximal 0,5 Mol%, vorzugsweise maximal 0,1 Mol% BaO. Besonders bevorzugt ist das Glas frei von BaO.

Das Glas kann in einer Ausführung vorteilhaft Anteile an ZnO enthalten, insbesondere 0,0 Mol% bis maximal oder weniger als 5,0 Mol%. Der Gehalt ist auf maximal 5,0 Mol% zu begrenzen, da sich die Komponente negativ auf die hydrolytische Beständigkeit des Glases auswirkt. Eine vorteilhafte Obergrenze kann auch maximal 3,0 Mol% oder maximal 2,0 Mol% sein. Wenn ZnO im Glas enthalten sein soll, kann mindestens 0,1 Mol% oder mindestens 0,2 Mol% oder mindestens 0,5 Mol% eine vorteilhafte Untergrenze sein. Bevorzugte Varianten des Glases können frei von ZnO sein.

Das Glas kann vorteilhaft Al₂O₃ enthalten, um die hydrolytische Beständigkeit zu erhöhen, den thermischen Ausdehnungskoeffizienten zu erhöhen und die Kristallisationsneigung zu verringern. Der Anteil von Al₂O₃ in der Glaszusammensetzung kann mindestens 3,0 Mol%, vorzugsweise mindestens 7,0 Mol%, bevorzugt mindestens 8,0 Mol%, besonders bevorzugt mindestens 9,0 Mol% betragen. Eine vorteilhafte Obergrenze kann maximal 20,0 Mol%, vorteilhaft maximal 17,0 Mol%, vorteilhaft maximal 16,0 Mol% sein. Die Obergrenze sollte nicht überschritten werden, weil ansonsten die Kristallisationsgefahr und/oder die Einglasungstemperatur steigt. Für manche vorteilhaften Varianten kann maximal 15,0 Mol% oder maximal 14,0 Mol% oder maximal 13,0 Mol% eine Obergrenze sein. Ein besonders vorteilhafter Bereich kann 7,0 Mol% bis 17,0 Mol% oder 7,0 bis < 15,0 Mol%, bevorzugt 8,0 bis 14,0 Mol% sein.

Gemäß bevorzugter Ausführungsformen sind die erfindungsgemäßen Gläser alkaliarm, und weiter bevorzugt alkalifrei, da Alkalimetallionen, die chemische Beständigkeit, insbesondere die hydrolytische Beständigkeit, des Glases verringern und damit schlecht für die Langzeit-Autoklavierbarkeit sind. Alkaliarm ist im Sinne der vorliegenden Erfindung so zu verstehen, dass die Summe der Alkalimetalloxide R₂O (Li₂O+Na₂O+K₂O+Cs₂O +Rb₂O) im Glas vorteilhaft maximal oder weniger als 3,0 Mol%, vorteilhaft maximal 2,0 Mol%, vorteilhaft maximal 1,0 Mol%, vorteilhaft maximal oder weniger als 0,5 Mol%, bevorzugt maximal oder weniger als 0,1 Mol% beträgt. Für den Fall, dass nur ein einziges Alkalimetalloxid im Glas enthalten ist, können die genannten Obergrenzen für jedes Alkalimetalloxid einzeln gelten. Falls zwei oder mehr Alkalimetalloxide enthalten sind, gelten die genannten Obergrenzen entsprechend für jeweiligen beliebigen Kombinationen. Besonders bevorzugte Varianten der Gläser sind bis auf übliche Verunreinigungen alkalifrei, d.h. frei von den Alkalimetalloxiden Li₂O und/oder Na₂O und/oder K₂O, insbesondere frei von Li₂O, Na₂O, K₂O, Cs₂O und/oder Rb₂O.

In Bezug auf die Glaszusammensetzung kann es vorteilhaft sein, wenn besondere Summen und/oder Verhältnisse der Glaskomponenten einzeln oder in Kombination erfüllt sind:
Die Summe der Komponenten SiO₂ + ZrO₂ + Nb₂O₅+TiO₂+Ta₂O₅ kann vorzugsweise im Bereich von > 10,5 bis 28,0 Mol% liegen. Damit kann einerseits die hydrolytische Beständigkeit verbessert und andererseits das Kristallisationsrisiko gering gehalten werden. Außerdem wird die Viskosität des Glases verbessert. Manche Varianten können eine vorteilhafte Untergrenze von 12,0 Mol% oder von 14,0 Mol% oder von 15,5 Mol% aufweisen und/oder vorteilhaft eine Obergrenze von 26,0 Mol% oder von 24,0 Mol% aufweisen. Bei manchen vorteilhaften Varianten kann der Bereich 15,5 Mol% bis 23,0 Mol% betragen.

Die Summe der Komponenten SiO₂ + B₂O₃ kann vorzugsweise im Bereich von > 40,5 bis 55,0 Mol% liegen. Damit kann einerseits die hydrolytische Beständigkeit verbessert und andererseits die Einglasungstemperatur niedrig gehalten werden. Manche Varianten können eine vorteilhafte Untergrenze von 42,0 Mol% oder von 43,0 Mol% aufweisen und/oder vorteilhaft eine Obergrenze von 53,0 Mol% oder von 51,0 Mol% aufweisen. Bei manchen vorteilhaften Varianten kann der Bereich 42,0 Mol% bis 53,0 Mol% betragen.

Das Verhältnis der Komponenten (SiO₂+Al₂O₃) / B₂O₃ kann vorzugsweise 0,70 bis 1,70 betragen. Dadurch kann die Einglasungstemperatur gering gehalten werden. Manche Varianten können eine vorteilhafte Untergrenze von 0,75 oder von 0,80 aufweisen und/oder vorteilhaft eine Obergrenze von 1,60 oder von 1,50 oder von 1,30 oder von 1,20 aufweisen. Bei manchen vorteilhaften Varianten kann der Bereich 0,80 bis 1,20 oder 0,85 bis 1,10 betragen.

Das Verhältnis der Komponenten (SiO₂+Al₂O₃) / (B₂O₃+RO) kann vorzugsweise 0,30 bis 0,90 betragen. Dadurch kann die Einglasungstemperatur gering gehalten werden und die Viskosität des Glases verbessert werden. Manche Varianten können eine vorteilhafte Untergrenze von 0,35 oder von 0,40 aufweisen und/oder vorteilhaft eine Obergrenze von 0,80 oder von 0,70 oder von 0,65 aufweisen. Bei manchen vorteilhaften Varianten kann der Bereich 0,35 bis 0,70 betragen.

Das Verhältnis der Komponenten (SiO₂+Al₂O₃+Nb₂O₅+ZrO₂+TiO₂+Ta₂O₅) / (B₂O₃+RO) kann vorzugsweise 0,35 bis 0,90 betragen. Damit kann einerseits die hydrolytische Beständigkeit verbessert und andererseits das Kristallisationsrisiko gering gehalten werden. Außerdem wird die Viskosität des Glases verbessert. Manche Varianten können eine vorteilhafte Untergrenze von 0,37 oder von 0,40 aufweisen und/oder vorteilhaft eine Obergrenze von 0,80 oder von 0,75 oder von 0,70 oder von 0,65 aufweisen. Bei manchen vorteilhaften Varianten kann der Bereich 0,35 bis 0,75 betragen.

Das Verhältnis der Komponenten (SiO₂) / (Nb₂O₅+ZrO₂+TiO₂+Ta₂O₅) beträgt mehr als 3,00. Ein vorteilhafter Bereich kann > 3,00 bis 55,00 sein. Dadurch liegt im Glas ein ausgewogenes Verhältnis der Komponenten vor, die im Rahmen der Erfindung die chemische Beständigkeit, insbesondere die hydrolytische Beständigkeit verbessern, aber auch das Kristallisationsrisiko erhöhen. Außerdem wird die Viskosität des Glases verbessert. Vorteilhaft kann das Verhältnis weniger als 55,00, bevorzugt weniger als 50,00, vorteilhaft weniger als 45,00 betragen. In manchen vorteilhaften Varianten kann das Verhältnis maximal 40,00 oder maximal 35,00 betragen.

Die Glaszusammensetzung ist bevorzugt frei von folgenden Elementen oder deren Verbindungen: Cr, Ni, Cd, Pb, Hg, As, Sb, Be, Ag, Sn, Cd, TI, da diese giftig sind und/oder ein Allergiepotential haben. Außerdem ist das Glas vorteilhaft alkaliarm, wobei die Summe der Alkalimetalloxide (Li₂O+Na₂O+K₂O+Cs₂O+Rb₂O), bevorzug weniger als 0,5 Mol% beträgt. Besonders bevorzugt ist das Glas alkalifrei (frei von R₂O).

Die Begriffe wie "Pb-frei bzw. bleifrei", "alkalifrei" oder ganz allgemein "frei von einer Komponente x" sind im Sinne der vorliegenden Erfindung so zu verstehen, dass diese Substanzen oder deren Oxide nicht als Komponente dem Glas beabsichtigt zugegeben sind und höchstens in Spuren oder als kleine Restmengen, d.h. höchstens als Verunreinigungen in dem Glas, vorliegen. Beispielsweise für Blei bedeutet dies, dass der Pb-Gehalt weniger als 1000 ppm beträgt. Vorteilhaft kann z.B. der Pb-Gehalt weniger als 500 ppm bevorzugt weniger als 100 ppm betragen. Beispielsweise kann für Li₂O, Na₂O, K₂O, Cs₂O, Rb₂O und/oder andere z.T. oben vorteilhaft mit "frei von" beschriebene Komponenten der Gehalt pro Komponente weniger als 1000 ppm oder weniger als 500 ppm, bevorzugt weniger als 100 ppm, bevorzugt weniger als 50 ppm betragen.

Gemäß einer Ausführungsform besteht das Glas zu mindestens 94,0 Mol%, vorzugsweise zu mindestens 95,0 Mol%, bevorzugt zumindest 97,0 Mol%, in manchen vorteilhaften Varianten zu mindestens 99,0 Mol% aus den Komponenten La₂O₃, B₂O₃, SiO₂, Al₂O₃, Nb₂O₅, ZrO₂, TiO₂, Ta₂O₅ und RO.

Gemäß einer vorteilhaften Variante ist das Glas frei von Komponenten, die in der Offenbarung nicht erwähnt sind.

In einer vorteilhaften Weiterbildung kann das Glas folgende Bestandteile in Mol% auf Oxidbasis aufweisen:

| | |
|---|---|
| B₂O₃ | 22,5 bis 36,0 |
| La₂O₃ | 1,0 bis 11,0 |
| Al₂O₃ | 7,0 bis 17,0 |
| SiO₂ | 10,5 bis 23,0 |
| MgO+CaO+SrO | 30,0 bis 39,5 |
| Nb₂O₅+ZrO₂+TiO₂+Ta₂O₅ | 0,1 bis 6,0 |

Weiterhin bevorzugt können im Glas - im Rahmen der oben genannten Grenzen für die Summe Nb₂O₅+ZrO₂+TiO₂+Ta₂O_{5 -} einzeln oder in jeder Kombination in Mol% folgende Komponenten enthalten sein:

| | |
|---|---|
| Nb₂O₅ | 0,0 bis 6,0 |
| ZrO₂ | 0,0 bis 6,0 |
| TiO₂ | 0,0 bis 6,0 |
| Ta₂O₅ | 0,0 bis 6,0 |

Weiterhin bevorzugt können im Glas - im Rahmen der oben genannten Grenzen für Al₂O₃ und für die Summe MgO+CaO+SrO - einzeln oder in jeder Kombination in Mol% folgende Komponenten enthalten sein:

| | |
|---|---|
| Al₂O₃ | 7,0 bis 17,0 |
| MgO | 0,0 bis 20,0 |
| CaO | 0,0 bis 20,0 |
| SrO | 0,0 bis 10,0 |

In einer vorteilhaften Weiterbildung kann das Glas einzeln oder in jeder Kombination innerhalb der genannten Grenzen folgende Komponenten in Mol% auf Oxidbasis aufweisen:

| | |
|---|---|
| B₂O₃ | 22,5 bis 36,0 |
| La₂O₃ | 1,0 bis 11,0 |
| Al₂O₃ | 7,0 bis 16,0 |

| | |
|---|---|
| SiO₂ | 10,5 bis 22,5 |
| ZrO₂ | 0,0 bis 5,0 |
| TiO₂ | 0,0 bis 5,0 |
| Nb₂O₅ | 0,0 bis 5,0 |
| MgO | 0,0 bis 18,0 |
| CaO | 0,0 bis 19,0 |
| SrO | 0,0 bis 8,0 |

Bevorzugt kann das Glas weniger als 3 Gew.-% an Füllstoffen enthalten. Füllstoffe werden häufig eingesetzt, um das Ausdehnungsverhalten, d.h. den CTE, des Glases an die verwendeten Metallkomponenten in der elektrischen Durchführung anzupassen. Vorzugsweise enthält das Glas weniger als 1 Gew.-% oder weniger als 0,5 Gew.-% an Füllstoffen. Bevorzugt ist das Glas frei von Füllstoffen. Das Glas, das einen definierten Gehalt bestimmter Komponenten, die als Beständigkeitsverstärker wirken, und einen gewissen Anteil an SiO₂ aufweist, hat im Rahmen der Offenbarung einen CTE, der auch ohne Füllstoffe an Titan und Titanlegierungen angepasst ist, so dass keine Füllstoffe zur Dehnungsanpassung benötigt werden.

In Bezug auf den thermischen Ausdehnungskoeffizienten (CTE) des Glases kann vorteilhaft vorgesehen sein, dass das Glas des Isolationsmaterials einen CTE(20°C; 300°C) aufweist, welcher im Bereich von 5,0 bis 13,0 ppm/K liegt, vorzugsweise im Bereich von 5,0 bis 10,5 ppm/K, vorzugsweise im Bereich von 6,0 bis 10,5 oder 6,0 bis 9,0 ppm/K liegt, besonders bevorzugt im Bereich von 6,5 bis 8,0 ppm/K liegt. Die Erfinder haben erkannt, dass es durch eine spezielle Auswahl der Glaskomponenten möglich ist, ein Glas mit hoher hydrolytischer Beständigkeit bereitzustellen, welches auch ohne Füllstoffe einen CTE aufweist, der für eine Glas-Metall-Durchführung mit Titan oder einer Titan-Legierung geeignet ist. Das war überraschend. Hierdurch kann insbesondere eine Materialabstimmung zu Titan oder Titanlegierungen erzielt werden, wodurch wiederum die Dichtheit der Durchführung, insbesondere hermetische Dichtheit, verbessert werden kann. Der CTE wird dilatometrisch in einer statischen Messung (mit einem Schubstangendilatometer) bestimmt gemäß ISO 7991:1987-12.

Das Glas des Isolationsmaterials kann vorteilhaft eine Dichte aufweisen, welche im Bereich von 2,50 bis 3,80 g/cm³ liegt, vorzugsweise im Bereich von 2,70 bis 3,70 g/cm³ liegt, besonders im Bereich von 2,80 bis 3,50 g/cm³ liegt. Die Dichte kann in bekannter Weise bestimmt werden, z.B. gemäß ASTM C693: 1993.

Ferner kann vorteilhaft vorgesehen sein, dass das Glas des Isolationsmaterials eine Glasübergangstemperatur T_{g} aufweist, welche niedriger ist als 750°C, vorzugsweise niedriger ist als 700 °C, vorzugsweise niedriger ist als 670 °C, besonders bevorzugt niedriger ist als 650 °C. Die Transformationstemperatur wird in bekannter Weise nach DIN ISO 7884-8:1998-02 bestimmt.

Insbesondere kann das Glas des Isolationsmaterials eine Glasübergangstemperatur T_{g} aufweisen, welche im Bereich von 500 bis 700 °C liegt, vorzugsweise im Bereich von 560 bis 670 °C liegt, besonders im Bereich von 600 bis 650 °C liegt. Grundsätzlich kann eine niedrigere Glas Glasübergangstemperatur T_{g} im Hinblick auf die Verarbeitung vorteilhaft sein.

Vorteilhaft kann das Glas des Isolationsmaterials eine sphärische Temperatur von maximal 850°C, vorzugsweise maximal 820°C, besonders bevorzugt maximal 790°C aufweisen.

Vorteilhaft kann das Glas des Isolationsmaterials eine Halbkugeltemperatur von maximal 900°C, vorzugsweise maximal 880°C, vorzugsweise maximal 870°C, bevorzugt maximal 860°C aufweisen.

Die Glaseigenschaften "sphärische Temperatur" und "Halbkugeltemperatur" wurden mittels der etablierten Methodik der Erhitzungsmikroskopie (EHM) mit einem Erhitzungsmikroskop EMI301 des Unternehmens "Hesse Instruments" mit der "EMI III Heating Microscope Software" bestimmt. Die Auswertung erfolgt automatisch, z.B. nach DIN 51730, indem das Schattenprofil einer Probe analysiert wird. Die Halbkugeltemperatur kennzeichnet diejenige Temperatur, bei der ein ursprünglich zylindrischer Probekörper zu einer halbkugelförmigen Masse zusammengeschmolzen ist. Die Halbkugeltemperatur des Glases entspricht in etwa der Höhe der Temperatur, bei der eine dichte Glas-Metall-Durchführung hergestellt werden kann, d.h. in etwa der Einglasungstemperatur. Die Einglasungstemperatur kann 5°C bis 70°C höher liegen als die Halbkugeltemperatur.

Der Grundkörper der Durchführung, auch als Außenleiter bezeichnet, umfasst Titan oder eine Titanlegierung, wobei das Material vorteilhaft ausgewählt ist aus Titan Grade 1, Titan Grade 2, Titan Grade 3, Titan Grade 4 oder Titan Grade 5, insbesondere eine TiAl6V4-Legierung. Titan und Titanlegierungen können einen thermischen Ausdehnungskoeffizienten CTE (20;300) aufweisen, welcher im Bereich von 8 bis 10 ppm/K, vorzugsweise im Bereich von 8,5 bis 9,5 ppm/K liegt.

Der elektrische Leiter der Durchführung kann ein Metall umfassen oder daraus bestehen. Bevorzugt ist das Metall ausgewählt aus Kovar, Molybdän, Nickel, Nickel-Eisen-Legierung, Titan, Titanlegierung, Platin, Platinlegierung (z.B. eine Pt/Ir-Legierung), Tantal, Tantallegierung, Niob, Nioblegierung.

Der elektrische Leiter kann einen thermischen Ausdehnungskoeffizienten CTE (20;300) aufweisen, welcher im Bereich von 5 bis 13 ppm/K, vorzugsweise im Bereich von 6 bis 10 ppm/K, vorzugsweise im Bereich von 7 bis 9 ppm/K liegt. In Verbindung mit einem Grundkörper mit oder aus Titan oder einer Titanlegierung kann durch entsprechende Wahl des Isolationsmaterials eine angepasste Einglasung bereitgestellt werden.

Bei einer angepassten Einglasung ist es bevorzugt, wenn eine Differenz der Ausdehnungskoeffizienten zwischen Grundkörper und Isolationsmaterial, vorzugsweise zwischen Grundkörper, Isolationsmaterial und Leiter, kleiner ist als 5%.

Insbesondere wird unter einer angepassten Durchführung verstanden, dass sich die Ausdehnungskoeffizienten im Wesentlichen um höchstens 1 * 10 ⁻⁶ 1/K unterscheiden, insbesondere im Wesentlichen gleich sind.

In einer vorteilhaften Alternative kann in Verbindung mit einem Grundkörper, der Titan oder eine Titanlegierung umfasst, eine Druckeinglasung bereitgestellt werden, wodurch die mechanische Robustheit verstärkt werden kann. Dabei wird ein thermischer Ausdehnungskoeffizient des Grundkörpers größer gewählt als ein thermischer Ausdehnungskoeffizient des Isolationsmaterials, so dass sich nach einer Temperaturbehandlung, bei der das Isolationsmaterial in der Durchgangsöffnung eingeglast wird, der Grundkörper stärker zusammenzieht als das Isolationsmaterial. Hierdurch werden dauerhaft Drucckräfte durch den Grundkörper auf das Isolationsmaterial ausgeübt. Diese spannen das Isolationsmaterial vor und sorgen für eine besonders beständige Abdichtung.

Entsprechend ist es bevorzugt, dass ein thermischer Ausdehnungskoeffizient des Grundkörpers größer ist als ein thermischer Ausdehnungskoeffizient des Isolationsmaterials. Besonders bevorzugt wird bei einer Druckeinglasung der thermische Ausdehnungskoeffizient des Grundkörpers mindestens 5%, bevorzugt mindestens 10%, besonders bevorzugt mindestens 20% und am meisten bevorzugt mindestens 50% größer gewählt als der thermische Ausdehnungskoeffizient des Isolationsmaterials.

Die Vorspannung für die Druckeinglasung wird im Wesentlichen durch den Unterschied der Ausdehnungskoeffizienten zwischen dem Material des Grundkörpers und dem Material des Isolationsmaterials bestimmt.

Soweit oben im Zusammenhang mit einer Druckeinglasung oder einer angepassten Einglasung für Materialien Werte für den Ausdehnungskoeffizienten genannt wurden, beziehen sich diese auf den im Zusammenhang mit Glas-Metall-Durchführungen üblicherweise angegebenen linearen thermischen Ausdehnungskoeffizienten α im Temperaturintervall 20-300°C.

Zur Herstellung einer Durchführung kann das Glas umfassende Isolationsmaterial bzw. ein Vorläufermaterial in Gestalt eines Formkörpers bereitgestellt werden. Der Formkörper kann beispielsweise die Form eines Hohlzylinders haben. Zur Ausbildung der elektrischen Durchführung wird der elektrische Leiter in das Innere dieses Hohlzylinders eingesetzt und dieser wird wiederum in eine Durchgangsöffnung eines Grundkörpers, der Titan oder eine Titanlegierung umfasst oder daraus besteht, eingesetzt. Durch eine Temperaturbehandlung wird anschließend der Metallstift in die Öffnung eingeglast, wobei das Isolationsmaterial, insbesondere das Glas, mit dem Material des Leiters und dem Material des Grundkörpers eine innige Verbindung eingeht, so dass eine Glas-Metall-Durchführung gebildet wird. Im Rahmen der Temperaturbehandlung schmilzt das Glas auf.

In einigen Fällen kann durch die Verwendung von Titan oder Titanlegierungen in Bezug auf chemische Reaktionen die Besonderheit auftreten, dass die Glaskomponente SiO₂ mit Titan zu Titansilizid reagiert, wodurch Ablösungserscheinungen an der Glas-Metall-Kontaktzone auftreten können. Dieses Problem kann insbesondere durch die vorstehenden Angaben zur Glaszusammensetzung vermindert oder vermieden werden. Insbesondere kann durch die genannten Angaben zu B₂O₃ diese Reaktion unterdrückt werden und zu einer TiB-Schicht führen, die zu chemisch und mechanisch stabileren Verbindungen zwischen einer titanumfassenden Komponente und dem Glas führt.

Grundsätzlich ist zu berücksichtigen, dass Titan bzw. Titanlegierungen stark reaktiv ist. Mit den erfindungsgemäßen Glaszusammensetzungen kann vermindert oder vermieden werden, dass das Titan(legierungen) beim Einglasen des Leiters in den Grundkörper (z.B. bei Einglasungstemperaturen von z.B. 700 bis 900°C) mit SiO₂ zu Titansilizid reagiert, und diese Reaktion z.B. von einer Blasenbildung am Interface begleitet wird. - Außerdem können mit den erfindungsgemäßen Gläsern die Einglasungstemperaturen geringer gewählt werden, um die Glas-Metall-Durchführungen unterhalb des Temperaturbereiches des α/β-Phasenübergangs von Titan herstellen zu können.

In einer vorteilhaften Variante kann die Durchführung genau einen elektrischen Leiter aufweisen, welcher sich durch das in der Durchgangsöffnung aufgenommene Isolationsmaterial hindurch erstreckt.

In einer vorteilhaften Variante kann die Durchführung eine Mehrzahl von elektrischen Leitern aufweisen, welche sich durch das in der Durchgangsöffnung aufgenommene Isolationsmaterial hindurch erstrecken, z.B. zumindest 2 elektrische Leiter, besonders bevorzugt zumindest 10 elektrische Leiter.

Der Grundkörper kann eine Mehrzahl von Durchgangsöffnungen mit jeweils darin aufgenommenen Isolationsmaterial umfassen, wobei sich jeweils zumindest ein, insbesondere genau ein, elektrischer Leiter durch das Isolationsmaterial einer Durchgangsöffnung erstreckt.

Der Titan oder eine Titanlegierung umfassende Grundkörper kann plattenförmig ausgebildet sein. Der Grundkörper kann eine erste und eine gegenüberliegende zweite Oberfläche aufweisen, wobei die Durchgangsöffnung eine Innenwandung bildet, welche die erste mit der zweiten Oberfläche verbindet. Der Grundkörper kann eine Ebene definieren welche parallel zu der ersten und/oder zweiten Oberfläche verläuft. Der Grundkörper kann entlang einer Richtung, welche parallel zu der ersten und/oder zweiten Oberfläche verläuft und/oder in der vorgenannten Ebene verläuft, eine Abmessung aufweisen, welche größer ist als der Durchmesser der Durchgangsöffnung, insbesondere mindestens zweimal so groß, insbesondere mindestens dreimal so groß ist.

Das in der Durchgangsöffnung befindliche Isolationsmaterial kann gegenüber der ersten und/oder zweiten Oberfläche des Grundkörpers zurückgesetzt sein. Mit anderen Worten kann das Isolationsmaterial derart in der Durchgangsöffnung aufgenommen sein, dass zu dem Grundkörper an der Stelle der Innenwandung eine Stufe besteht. Alternativ kann es auch an einer Oberfläche des Grundkörpers oder an beiden Oberflächen des Grundkörpers bündig sein oder sogar darüber hinausragen.

Der Leiter kann gegenüber der ersten und/oder zweiten Oberfläche des Grundkörpers hervorstehen.

Ein weiterer Aspekt der Erfindung betrifft ein Glas, insbesondere für eine elektrische Durchführung mit einem Grundkörper aus Titan oder einer Titanlegierung nach dem ersten Aspekt der Erfindung, wobei das Glas ein Lanthan-Borat-Glas ist, das folgende Komponenten umfasst (in Mol% auf Oxidbasis):

| | | | |
|---|---|---|---|
| B₂O₃ | 22,0 | - | 37,0 |
| La₂O₃ | 1,0 | - | 12,0 |
| SiO₂ | 10,5 | - | 23,0 |
| RO (MgO+CaO+SrO) | 29,0 | - | 45,0 |
| Nb₂O₅+ZrO₂+TiO₂+Ta₂O₅ | 0,1 | - | 6,0 |
| SiO₂/(Nb₂O₅+ZrO₂+TiO₂+Ta₂O₅) | > 3,00 | | |

wobei das Glas bei Bestimmung der hydrolytischen Beständigkeit gemäß DIN ISO 720:2021-12 einen Na₂O Äquivalenzwert von < 1250 µg/g aufweist.

Die genannten Komponenten sind Glaskomponenten. D.h. mindestens eine Komponente aus (Nb₂O₅+ ZrO₂ +TiO₂ +Ta₂O₅) ist Bestandteil des erschmolzenen Glases.

Einzelheiten zu den Vorteilen der erfindungsgemäßen Glaszusammensetzung, zu deren vorteilhaften Weiterbildungen, zu vorteilhaften chemischen und physikalischen Eigenschaften etc. wurden oben bereits im Zusammenhang mit der Beschreibung des ersten Aspektes der Erfindung beschrieben. Diese Offenbarung wird vollumfänglich mit in die Beschreibung des weiteren Aspektes der Erfindung mit aufgenommen. Zur Vermeidung von Wiederholungen wird darauf verwiesen.

Das Glas und eine damit hergestellte elektrische Durchführung kann in elektrischen Geräten, medizinischen Geräten, insbesondere im Zusammenhang mit autoklavierbaren Durchführungen mit Metallkomponenten aus Titan oder einer Titan-Legierung eingesetzt werden. Ein vorteilhaftes Einsatzgebiet sind Endoskope und chirurgische Instrumente, die zum Untersuchen, Operieren und Behandeln verwendet werden und nur kurzzeitig mit dem Körper Kontakt haben, aber dafür öfter nach entsprechender Sterilisation wiederverwendet werden können, z.B. Geräte in der Roboterchirurgie. Ein Einsatzgebiet sind auch implantierbare medizinische Geräte oder am Körper tragbare Geräte, z.B. Wearables. Da Komponenten aus Titan bzw. Titanlegierungen aufgrund der besonderen Festigkeit, Beständigkeit und des geringen Gewichts häufig in Gebieten wie Luft- und Raumfahrt, Rennsport usw. zum Einsatz kommen, kann das Glas und damit hergestellte Fügeverbindungen und Durchführungen auch dort zum Einsatz kommen.

Ein weiterer Aspekt der Erfindung betriff somit die Verwendung einer erfindungsgemäßen Durchführung oder eines erfindungsgemäßen Glases in einer Durchführung oder Fügeverbindung mit einer Metallkomponente aus Titan oder einer Titan-Legierung in den Bereichen medizinische Geräte, Wearables, Luft- und Raumfahrt, Rennsport.

Es versteht sich, dass die vorstehend genannten und die nachstehend noch zu erläuternden Merkmale der Erfindung, die mehrerer Aspekte umfasst, nicht nur in der jeweils angegebenen Kombination, sondern auch in anderen Kombinationen verwendbar sind, ohne den Rahmen der Erfindung zu verlassen.

Die Erfindung wird nachfolgend anhand von Figuren und einigen Ausführungsbeispielen näher beschrieben. Dabei zeigen:
Fig. 1: eine schematische Darstellung einer Durchführung gemäß einer ersten Ausführungsform,
Fig. 2: eine schematische Darstellung einer Durchführung gemäß einer zweiten Ausführungsform,
Fig. 3: eine schematische Darstellung einer Durchführung gemäß einer dritten Ausführungsform.

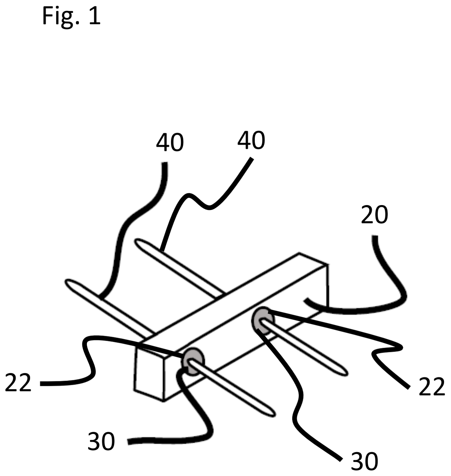

Bezugnehmend auf Fig. 1 weist eine Langzeit-autoklavierbare Durchführung einen äußeren Grundkörper 20 auf, durch welchen eine oder mehrere Durchgangsöffnungen 22 (hier zwei) verlaufen, wobei in einer Durchgangsöffnung 22 jeweils ein Isolationsmaterial 30 umfassend oder bestehend aus Glas eingebracht ist, durch welches sich zumindest ein elektrischer Leiter 40 erstreckt. Der Leiter kann dabei aus dem Isolationsmaterial einseitig oder beidseitig herausragen (hier beidseitig). Die gezeigte Durchführung weist zwei Innenleiter (Pins) auf und kann daher als eine 2-polige Durchführung bezeichnet werden. Es ist möglich, dass der Grundkörper 20 als Außenleiter dient und somit einen weiteren elektrischen Leiter bildet. Selbstverständlich sind auch Durchführungen mit nur einem Innenleiter (Pin) möglich, d.h. einfache Durchführungen oder auch 1-polige Durchführungen.

Der Grundkörper 20 ist in diesem Beispiel aus Titan, alternativ könnte es sich um eine Titanlegierung handeln. Die Leiter 40 bestehen hier ebenfalls aus Titan. Alternativ könnten sie jedoch auch aus einem anderen biokompatiblen Material bestehen, beispielsweise Titanlegierung, Kovar, Molybdän, Nickel-Eisen-Legierung, Nickel, Tantal, Tantallegierung, Niob, Nioblegierung, Platin, Platinlegierung. Das Isolationsmaterial 30 besteht aus einem erfindungsgemäßen Erdalkalimetalloxid-haltigen Lanthan-Borat-Glas.

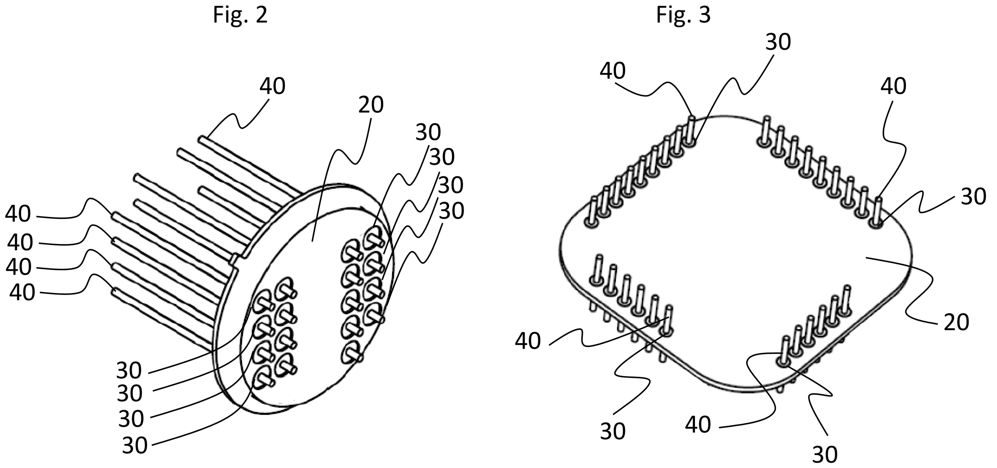

Bezugnehmend auf Fig. 2 und 3 kann eine Langzeit-autoklavierbare Durchführung auch eine Vielzahl von Innenleitern (Pins) aufweisen, so dass z.B. eine 17-polige Durchführung (Fig. 2) oder auch eine 30-polige Durchführung (Fig. 3) vorgesehen sein kann. Bei den gezeigten Steckverbindern erstreckt sich jeder einzelne Innenleiter 40 durch das Isolationsmaterial einer einzelnen Durchgangsöffnung 22. Es ist aber auch möglich, dass sich eine Mehrzahl oder Vielzahl elektrischer Leiter durch dasselbe Isolationsmaterial derselben Durchgangsöffnung 22 erstrecken.

### Beispiele

Aus herkömmlichen Rohstoffen wurden bei Temperaturen oberhalb von 1350°C Gläser mit Zusammensetzungen gemäß Tabellen 1 und 2 in beheizten Pt-Tiegeln bzw. Pt/Ir-Tiegeln erschmolzen. Die Schmelze wurde mehr als 20 Minuten bei dieser Temperatur gehalten, zur Homogenisierung gerührt und zu Gussblöcken gegossen.

Neben der Zusammensetzung wurden folgende Kenngrößen und Eigenschaften der Gläser der erfindungsgemäßen Ausführungsbeispiele (Bsp.) und der Vergleichsbeispiele (Vgl.-Bsp.) an massiven Proben ermittelt nach den Methoden wie oben beschrieben: CTE (20;300), Dichte, T_{g}.

Zur Herstellung eines Glaspulvers kann die Schmelze durch wassergekühlte Metallwalzen gegeben und das Glasband (Ribbon) anschließend aufgemahlen werden.

Aus den reinen Pulvern wurden in bekannter Weise Presslinge hergestellt und zur Charakterisierung des Erweichungsverhaltens, Schmelzverhaltens etc. der Gläser charakteristische Punkte mittels Erhitzungsmikroskopie (EHM) ermittelt nach der Methode wie oben beschrieben: sphärische Temperatur, Halbkugeltemperatur.

Aus den Glaspulvern wurden in bekannter Weise hohlzylinderförmige Formkörper hergestellt für die Herstellung von elektrischen Durchführungen. Zur Ausbildung einer elektrischen Durchführung wurde jeweils ein elektrischer Leiter aus Titan in das Innere eines Hohlzylinders eingesetzt und dieser wird wiederum in eine Durchgangsöffnung eines Grundkörpers aus Titan eingesetzt. Durch eine Temperaturbehandlung wurde anschließend der Metallstift in die Öffnung eingeglast, wobei das Glas, aufschmolz und mit dem Material des Leiters und dem Material des Grundkörpers eine innige Verbindung einging, so dass jeweils eine Glas-Metall-Durchführung gebildet wurde. Die Temperatur, bei der erfolgreich hermetisch dichte Durchführungen hergestellt werden können, ist die Einglasungstemperatur.

Nach Herstellung der Durchführungen, d.h. vor dem ersten Autoklavieren, wurde jeweils der Isolationswiderstand an den Durchführungen mit eingeglasten Leitern gemessen nach der Methode (MIL-STD-883, Meth. 1003) wie eingangs beschrieben. Dabei betrug jeweils der Durchmesser der Durchgangsöffnung im Grundkörper 1,8 mm, der Durchmesser des Leiters 0,5 mm und die Einglasungslänge 3,0 mm.

Anschließend wurde an den Durchführungen jeweils die Langzeit-Autoklavierungsbeständigkeit ermittelt wie folgt: Die Durchführungen wurden in einer Petrischale auf einem Gitter im Autoklaven platziert. Dies ist auf mehreren Schienen möglich. Daraufhin wurde der fest programmierte Autoklavierungsablauf mit den Parametern 135,5°C, 2,16 bar, 20 min gestartet. Die Durchführungen verblieben dort über mindestens 300 Zyklen. Jeder Zyklus umfasst dabei folgende Phasen: Entfernen von Luft aus der Sterilisationskammer; Dampferzeugung; Sterilisationsphase (bei 135,5°C und 2,16 bar Druck für 20 min); Vakuumtrocknen; Entfernung von Brauchwasser aus dem Kreislauf; Druckausgleich in der Sterilisationskammer bis zum Luftdruck und aktive Kühlung auf Raumtemperatur (Zyklusende). Nach den ersten 300 Zyklen wurde jeweils der Isolationswiderstand an den Durchführungen wieder gemessen. Wenn der Isolationswiderstand noch mindestens 1x10⁹ Ohm betrug, wurden die Durchführungen für weitere 300 Zyklen autoklaviert und danach der Isolationswiderstand erneut gemessen. Bei Durchführungen mit den erfindungsgemäßen Gläsern (Bsp. 1-4, 12-14) wurden die Tests nach 1200 Zyklen abgebrochen, da der Isolationswiderstand immer noch oberhalb des geforderten Mindestwiderstandes lag.

Mit bekannten Gläsern (Vgl.-Bsp. A-C), die in Durchführungen in implantierbaren Geräten zum Einsatz kommen können, wurden ebenfalls entsprechende Durchführungen hergestellt und unter denselben Bedingungen getestet. Bereits nach 300 Autoklavierungszyklen wurden die Tests abgebrochen, da der gemessene Isolationswiderstand weniger als 1*10⁹ Ohm betrug.

Außerdem wurde nach Abschluss bzw. Abbruch der Autoklavierungszyklen die Helium-Leckrate an den Durchführungen bestimmt.

Tabelle 1 zeigt 14 Ausführungsbeispiele (Bsp.) der Erfindung, während in Tabelle 2 Vergleichsbeispiele (Vgl. Bsp.) aufgeführt sind.

**Tabelle 1: Ausführungsbeispiele (Bsp. 1 bis 7, in Mol%)**

| **Bsp. Nr.** | **1** | **2** | **3** | **4** | **5** | **6** | **7** |
|---|---|---|---|---|---|---|---|
| B₂O₃ | 30,3 | 29,5 | 30,2 | 30,2 | 35,0 | 22,5 | 28,2 |
| CaO | 18,8 | 16,0 | 15,6 | 14,7 | 14,7 | 15,0 | 14,5 |
| MgO | 15,1 | 13,7 | 14,6 | 13,7 | 13,6 | 12,0 | 13,7 |
| SrO | 5,5 | 5,3 | 5,5 | 5,0 | 4,5 | 5,3 | 5,0 |
| La₂O₃ | 5,0 | 4,9 | 5,0 | 5,0 | 4,5 | 5,3 | 10,0 |
| SiO₂ | 10,6 | 17,6 | 16,5 | 16,5 | 16,0 | 22,0 | 16,5 |
| Al₂O₃ | 14,6 | 12,0 | 12,0 | 12,0 | 10,8 | 12,5 | 11,8 |
| Nb₂O₅ | 0,2 | 1,0 | | | 1,0 | 2,0 | |
| ZrO₂ | | | 0,6 | 3,0 | | 1,8 | 0,2 |
| TiO₂ | | | | | | 1,5 | 0,2 |
| Summe | 100,0 | 100,0 | 100,0 | 100,0 | 100,0 | 100,0 | 100,0 |
| | | | | | | | |
| SiO₂ + B₂O₃ | 40,9 | 47,1 | 46,7 | 46,7 | 51,0 | 44,5 | 44,7 |
| RO | 39,4 | 35,0 | 35,7 | 33,4 | 32,8 | 32,3 | 33,2 |
| ZrO₂+Nb₂O₅+TiO₂+Ta₂O₅ | 0,2 | 1,0 | 0,6 | 3,0 | 1,0 | 5,3 | 0,4 |
| SiO₂+Nb₂O₅+ZrO₂+TiO₂+Ta₂O₅ | 10,8 | 18,6 | 17,1 | 19,5 | 17,0 | 27,3 | 16,9 |
| (SiO₂+Al₂O₃) / B₂O₃ | 0,83 | 1,00 | 0,94 | 0,94 | 0,77 | 1,53 | 1,00 |
| (SiO₂+Al₂O₃) / (B₂O₃+RO) | 0,36 | 0,46 | 0,43 | 0,45 | 0,40 | 0,63 | 0,46 |
| (SiO₂+AL₂O₃) / (B₂O₃+La₂O₃) | 0,71 | 0,86 | 0,81 | 0,81 | 0,68 | 1,24 | 0,74 |
| (SiO₂+Al₂O₃+Nb₂O₅+ZrO₂+TiO₂ +Ta₂O₅) / (B₂O₃+RO) | 0,36 | 0,47 | 0,44 | 0,50 | 0,41 | 0,73 | 0,47 |
| (SiO₂)/(Nb₂O₅+ZrO₂+TiO₂+Ta₂O₅) | 53,00 | 17,60 | 27,50 | 5,50 | 16,00 | 4,15 | 41,25 |
| | | | | | | | |

| **Eigenschaften** | | | | | | | |
|---|---|---|---|---|---|---|---|
| CTE (20-300), ppm/K | 7,6 | 7,2 | 7,4 | 7,1 | 6,8 | 6,8 | 8,0 |
| T_{g}, °C | 626 | 632 | 626 | 640 | 642 | 642 | 648 |
| Density, g/cm3 | 3,21 | 3,20 | 3,19 | 3,22 | 3,04 | 3,04 | 3,57 |
| Sphärische Temp., °C | 750 | 765 | 764 | 779 | 779 | | |
| Halbkugeltemp., °C | 843 | 847 | 831 | 832 | 837 | | |
| Einglasungstemp., °C | 880 | 850 | 850 | 850 | | | |
| | | | | | | | |
| Autoklav. Zyklen/AZ (Anzahl) | 1200 | 1200 | 1200 | 1200 | | | |
| Isolationswiderstand, Ohm (vor dem Autoklavieren) | 2,00* 10¹¹ | 1,00* 10¹¹ | 1,00* 10¹¹ | >1* 10¹¹ | | | |
| Isolationswiderstand, Ohm (nach AZ-Anzahl) | 2,00* 10¹¹ | 2,00* 10¹⁰ | 5,00* 10⁹ | 1,00* 10¹¹ | | | |
| Dichtheit (Helium-Leckrate), mbar \| s⁻¹ | <1* 10⁻⁸ | <1* 10⁻⁸ | <1* 10⁻⁸ | <1* 10⁻⁸ | | | |
| Äquivalent Na₂O, µg/g (nach DIN ISO720) | 1240 | 786 | 842 | 790 | | | |

**Tabelle 1: Ausführungsbeispiele (Bsp. 8 bis 14, in Mol%)**

| **Bsp. Nr.** | **8** | **9** | **10** | **11** | **12** | **13** | **14** |
|---|---|---|---|---|---|---|---|
| B₂O₃ | 33,0 | 30,0 | 30,0 | 30,0 | 29,7 | 30,0 | 30,0 |
| CaO | 16,0 | 15,0 | 15,0 | 15,0 | 14,6 | 16,3 | 15,0 |
| MgO | 15,0 | 14,0 | 14,0 | 9,3 | 13,6 | 14,0 | 12,3 |
| SrO | 5,5 | 5,5 | 5,5 | 8,5 | 4,5 | 5,6 | 4,3 |
| La₂O₃ | 2,0 | 4,5 | 4,5 | 5,0 | 4,5 | 4,9 | 4,9 |
| SiO₂ | 12,0 | 14,0 | 14,0 | 13,0 | 21,0 | 18,6 | 17,6 |
| Al₂O₃ | 13,0 | 13,0 | 13,0 | 15,0 | 11,5 | 9,5 | 12,0 |
| Nb₂O₅ | | | | 3,5 | | 1,2 | 4,0 |
| ZrO₂ | 1,5 | | 3,5 | 0,8 | 0,6 | | |
| TiO₂ | 2,0 | 4,0 | 0,5 | | | | |
| Summe | 100,0 | 100,0 | 100,0 | 100,0 | 100,0 | 100,0 | 100,0 |
| | | | | | | | |
| SiO₂ + B₂O₃ | 45,0 | 44,0 | 44,0 | 43,0 | 50,7 | 48,6 | 47,6 |
| RO | 36,5 | 34,5 | 34,5 | 32,8 | 32,7 | 35,9 | 31,6 |
| ZrO₂+Nb₂O₅+TiO₂+Ta₂O₅ | 3,5 | 4,0 | 4,0 | 4,3 | 0,6 | 1,2 | 4,0 |
| SiO₂+Nb₂O₅+ZrO₂+TiO₂+Ta₂O₅ | 15,5 | 18,0 | 18,0 | 17,3 | 21,6 | 19,8 | 21,6 |
| (SiO₂+Al₂O₃) / B₂O₃ | 0,76 | 0,90 | 0,90 | 0,93 | 1,09 | 0,94 | 0,99 |
| (SiO₂+Al₂O₃) / (B₂O₃+RO) | 0,36 | 0,42 | 0,42 | 0,45 | 0,52 | 0,43 | 0,48 |
| (SiO₂+Al₂O₃) / (B₂O₃+La₂O₃) | 0,71 | 0,78 | 0,78 | 0,80 | 0,95 | 0,81 | 0,85 |
| (SiO₂+Al₂O₃+Nb₂O₅+ZrO₂+TiO₂ +Ta₂O₅) / (B₂O₃+RO) | 0,41 | 0,48 | 0,48 | 0,51 | 0,53 | 0,44 | 0,55 |
| (SiO₂)/(Nb₂O₅+ZrO₂+TiO₂+Ta₂O₅) | 3,43 | 3,50 | 3,50 | 3,02 | 35,00 | 15,50 | 4,40 |
| | | | | | | | |

| **Eigenschaften** | | | | | | | |
|---|---|---|---|---|---|---|---|
| CTE (20-300), ppm/K | 7,0 | 7,2 | 7,2 | 7,2 | 6,8 | 7,5 | 6,9 |
| T_{g}, °C | 627 | 634 | 634 | 640 | 639 | 632 | 630 |
| Density, g/cm3 | 2,87 | 3,11 | 3,10 | 3,41 | 3,11 | 3,23 | 3,28 |
| Sphärische Temp., °C | | | | | 785 | 767 | 780 |
| Halbkugeltemp., °C | | | | | 850 | 836 | 858 |
| Einglasungstemp., °C | | | | | 850 | 850 | 850 |
| | | | | | | | |
| Autoklav. Zyklen/AZ (Anzahl) | | | | | 1200 | 1200 | 1200 |
| Isolationswiderstand, Ohm (vor dem Autoklavieren) | | | | | >1* 10¹¹ | >1* 10¹¹ | >1* 10¹¹ |
| Isolationswiderstand, Ohm (nach AZ-Anzahl) | | | | | 1,00* 10¹¹ | 1,00* 10¹⁰ | 1,00* 10¹¹ |
| Dichtheit (Helium-Leckrate), mbar \| s⁻¹ | | | | | <1* 10⁻⁸ | <1* 10⁻⁸ | <1* 10⁻⁸ |
| Äquivalent Na₂O, µg/g (nach DIN ISO720) | | | | | 700 | 840 | 600 |

**Tabelle 2: Vergleichsbeispiele (Vgl.-Bsp. A bis C, in Mol%)**

| **Vgl.-Bsp. Nr.** | **A** | **B** | **C** |
|---|---|---|---|
| B₂O₃ | 30,3 | 26,6 | 51,0 |
| CaO | 18,8 | 4,4 | 17,0 |
| MgO | 15,1 | | |
| SrO | 5,5 | | |
| La₂O₃ | 5,0 | | 15,0 |
| SiO₂ | 10,1 | 50,7 | |
| Al₂O₃ | 15,2 | 5,1 | 5,0 |
| Nb₂O₅ | | | |
| ZrO₂ | | | |
| TiO₂ | | | 12,0 |
| Na₂O | | 13,2 | |
| Summe | 100,0 | 100,0 | 100,0 |
| | | | |
| SiO₂ + B₂O₃ | 40,4 | 77,3 | 15,0 |
| RO | 39,4 | 4,4 | |
| ZrO₂+Nb₂O₅+TiO₂+Ta₂O₅ | | | 12,0 |
| SiO₂+Nb₂O₅+ZrO₂+TiO₂+Ta₂O₅ | 10,1 | 50,7 | 12,0 |
| (SiO₂+Al₂O₃) / B₂O₃ | 0,83 | 2,10 | 1,33 |
| (SiO₂+Al₂O₃) / (B₂O₃+RO) | 0,36 | 1,80 | 1,00 |
| (SiO₂+Al₂O₃) / (B₂O₃+La₂O₃) | 0,71 | 2,10 | 1,03 |
| (SiO₂+Al₂O₃+Nb₂O₅+ZrO₂+TiO₂ +Ta₂O₅) / (B₂O₃+RO) | 0,36 | 1,80 | 1,00 |
| (SiO₂)/(Nb₂O₅+ZrO₂+TiO₂ +Ta₂O₅) | | | |
| | | | |

| **Eigenschaften** | | | |
|---|---|---|---|
| CTE (20-300), ppm/K | 7,8 | 7,4 | 7,7 |
| T_{g}, °C | 620 | 535 | 653 |
| Density, g/cm3 | 3,20 | 2,41 | 3,83 |
| Sphärische Temp., °C | 746 | 716 | 729 |
| Halbkugeltemp., °C | 845 | 799 | 800 |
| Einglasungstemp., °C | 900 | 820 | 870 |
| | | | |
| Autoklav. Zyklen/AZ (Anzahl) | 300 | 300 | 300 |
| Isolationswiderstand, Ohm (vor dem Autoklavieren) | 1,00*10¹² | 1,00*10¹¹ | 1,00*10⁶ |
| Isolationswiderstand, Ohm (nach AZ-Anzahl) | 5,00*10⁸ | 1,00*10⁶ | 1,00*10⁶ |
| Dichtheit (He-Leckrate), mbar \| s⁻¹ | >1*10⁻⁸ | >1*10⁻⁸ | >1*10⁻⁸ |
| Äquivalent Na₂O, µg/g (nach DIN ISO720) | 1200 | 1800 | 992 |

Die Bsp. 2 bis 4, 12 bis 14 zeigen im Vergleich mit den Vgl.-Bsp. A bis C niedrigere Na₂O Äquivalenzwerte, d.h. die erfindungsgemäßen Gläser weisen eine bessere hydrolytische Beständigkeit auf. Dabei beträgt der Na₂O Äquivalenzwert bei den Bsp. 2 bis 4 und 12 bis 14 weniger als 930 µg/g, d.h. diese Gläser fallen in die Klasse 3 nach DIN ISO 720. Die verbesserte hydrolytische Beständigkeit geht zurück auf einen für Glas-Metall-Durchführung mit Titan oder einer Titanlegierung relativ hohen Anteil an SiO₂ und einen bestimmten Anteil an Beständigkeitsverstärkern, ausgewählt aus der Gruppe bestehend aus Nb₂O₅, ZrO₂, TiO₂, Ta₂O₅, wobei SiO₂ und Beständigkeitsverstärker in einem ausgewogenen Verhältnis zueinander vorliegen.

Mit den erfindungsgemäßen Gläsern können Langzeit-autoklavierungsbeständige Durchführungen in Grundkörper aus Titan bzw. einer Titanlegierung bereitgestellt werden. Während bei den Vgl.-Bsp. A und B bereits nach 300 Autoklavierungszyklen der geforderte Mindest-Isolationswiderstand von 1*10⁹ Ohm nicht mehr erreicht wird, können mit den Gläsern von Bsp. 1 bis 4, 12 bis 14 Durchführungen hergestellt werden, deren Isolationswiderstand nach 1200 Autoklavierungszyklen immer noch höher als 1*10⁹ Ohm liegt und die Helium-Leckrate weniger als 1*10⁻⁸ mbar*l/s beträgt, d.h. die erfindungsgemäßen Durchführung bleiben auch nach mehr als 300 (hier nach 1200) Autoklavierungszyklen hermetisch dicht, anders als die Vergleichsdurchführungen mit den Gläsern der Vgl.-Bsp. A bis C.

Außerdem weisen die erfindungsgemäßen Gläser auch ohne Füllstoffe einen CTE (20;300) auf, der passend ist für die Herstellung hermetisch dichter Durchführungen mit Grundkörpern aus Titan bzw. Titanlegierungen und Leitern aus den oben definierten Materialien.

Das Erweichungs- und Schmelzverhalten ist bei den erfindungsgemäßen Gläsern derart optimiert, dass die Einglasung in Titan oder Titanlegierungen nicht zu weit oberhalb oder besonders bevorzugt unterhalb des Temperaturbereiches des α/β-Phasenübergangs von Titan durchgeführt werden kann. Die Halbkugeltemperatur der Bsp. 1 bis 4, 12 bis 14 beträgt weniger als 870°C, so dass die Einglasungstemperatur entsprechend niedrig gewählt werden kann, insbesondere < 930°C, vorzugsweise < 900°C.

## Patentansprüche

1. Elektrische Durchführung umfassend:
einen Grundkörper mit zumindest einer durch den Grundkörper verlaufenden Durchgangsöffnung,
ein Isolationsmaterial, welches in der durch den Grundkörper verlaufenden Durchgangsöffnung aufgenommen ist, wobei das Isolationsmaterial aus Glas besteht oder ein Glas umfasst,
zumindest einen elektrischen Leiter, welcher sich durch das in der Durchgangsöffnung aufgenommene Isolationsmaterial hindurch erstreckt, wobei der Grundkörper Titan oder eine Titanlegierung umfasst,
**dadurch gekennzeichnet, dass**
das Glas ein Lanthan-Borat-Glas ist, das folgende Komponenten (in Mol-% auf Oxidbasis) umfasst
| | | | |
|---|---|---|---|
| B₂O₃ | 22,0 | - | 37,0 |
| La₂O₃ | 1,0 | - | 12,0 |
| SiO₂ | 10,5 | - | 23,0 |
| RO (MgO+CaO+SrO) | 29,0 | - | 45,0 |
| Nb₂O₅+ZrO₂+TiO₂+Ta₂O₅ | 0,1 | - | 6,0 |
| SiO₂/(Nb₂O₅+ZrO₂+TiO₂+Ta₂O₅) | > 3,00 | | |
wobei das Glas bei Bestimmung der hydrolytischen Beständigkeit gemäß DIN ISO 720:2021-12 einen Na₂O Äquivalenzwert von < 1250 µg/g aufweist, wobei die Durchführung mit einem solchen Glas Langzeit-autoklavierungsbeständig ist, und
wobei ein Isolationswiderstand der Durchführung nach 300 Autoklavierungen mindestens 1*10⁹ Ohm beträgt.

2. Elektrische Durchführung nach Anspruch 1, wobei die Durchführung nach 300 Autoklavierungen eine Hermetizität aufweist, die **gekennzeichnet ist durch** eine Helium-Leckrate von weniger als 1*10⁻⁸ mbar | s⁻¹.

3. Elektrische Durchführung nach Anspruch 1 oder 2, wobei das Glas einzeln oder in jeder Kombination folgende Komponenten in Mol% auf Oxidbasis umfasst:
| | | | |
|---|---|---|---|
| Nb₂O₅ | 0,0 | - | 6,0 |
| ZrO₂ | 0,0 | - | 6,0 |
| TiO₂ | 0,0 | - | 6,0 |
| Ta₂O₅ | 0,0 | - | 6,0 |

4. Elektrische Durchführung nach mindestens einem der vorstehenden Ansprüche, wobei die Summe der Komponenten SiO₂ + ZrO₂ + Nb₂O₅+TiO₂+Ta₂O₅ im Bereich von > 10,5 bis 28,0 Mol% liegt.

5. Elektrische Durchführung nach mindestens einem der vorstehenden Ansprüche, wobei das Glas einzeln oder in jeder Kombination folgende Komponenten in Mol% auf Oxidbasis umfasst:
| | | | |
|---|---|---|---|
| Al₂O₃ | 7,0 | - | 17,0 |
| MgO | 0,0 | - | 20,0 |
| CaO | 0,0 | - | 20,0 |
| SrO | 0,0 | - | 10,0 |

6. Elektrische Durchführung nach mindestens einem der vorstehenden Ansprüche, wobei der SiO₂-Gehalt im Glas mindestens 12,0 Mol%, vorzugsweise mehr als 15,0 Mol% beträgt und/oder wobei das Glas 7,0 bis < 15,0 Mol%, vorzugsweise 8,0 bis 14,0 Mol% an Al₂O₃ enthält.

7. Elektrische Durchführung nach mindestens einem der vorstehenden Ansprüche, wobei das Glas mindestens eine Bedingung erfüllt:
- maximal 1,5 Mol% BaO, vorzugsweise frei von BaO
- < 3,0 Mol% Alkalimetalloxide (R₂O), vorzugsweise frei von R₂O.

8. Elektrische Durchführung nach mindestens einem der vorstehenden Ansprüche, wobei das Glas mindestens eine der folgenden Bedingungen erfüllt:
- einen thermischen Ausdehnungskoeffizienten (20°C; 300°C), welcher im Bereich von 5,0 bis 13,0 ppm/K, vorzugsweise im Bereich von 6,0 bis 10,5 ppm/K liegt,
- eine Glasübergangstemperatur T_{g}, welche niedriger ist als 750°C, vorzugsweise niedriger ist als 700 °C,
- eine Halbkugeltemperatur von maximal 900°C, vorzugsweise maximal 870°C.

9. Elektrische Durchführung nach mindestens einem der vorstehenden Ansprüche, wobei das Material des Grundkörpers ausgewählt ist aus Titan Grade 1, Titan Grade 2, Titan Grade 3, Titan Grade 4 oder Titan Grade 5, insbesondere eine TiAl6V4-Legierung, und/oder
wobei der elektrische Leiter ein Material ausgewählt aus Kovar, Molybdän, Nickel, Nickel-Eisen-Legierung, Titan, Titanlegierung, Platin, Platinlegierung (z.B. eine Pt/Ir-Legierung), Tantal, Tantallegierung, Niob, Nioblegierung umfasst oder daraus besteht.

10. Glas, insbesondere für eine elektrische Durchführung mit einem Grundkörper aus Titan oder einer Titanlegierung nach mindestens einem der vorhergehenden Ansprüche 1 bis 9, wobei das Glas ein Lanthan-Borat-Glas ist, das folgende Komponenten (in Mol-% auf Oxidbasis) umfasst
| | | | |
|---|---|---|---|
| B₂O₃ | 22,0 | - | 37,0 |
| La₂O₃ | 1,0 | - | 12,0 |
| SiO₂ | 10,5 | - | 23,0 |
| RO (MgO+CaO+SrO) | 29,0 | - | 45,0 |
| Nb₂O₅+ZrO₂+TiO₂+Ta₂O₅ | 0,1 | - | 6,0 |
| SiO₂/(Nb₂O₅+ZrO₂+TiO₂+Ta₂O₅) | > 3,00, | | |
wobei das Glas bei Bestimmung der hydrolytischen Beständigkeit gemäß DIN ISO 720:2021-12 einen Na₂O Äquivalenzwert von < 1250 µg/g aufweist.

11. Verwendung einer Durchführung nach mindestens einem der Ansprüche 1 bis 9 oder eines Glases nach Anspruch 10 in Durchführungen oder Fügeverbindungen mit einer Metallkomponente aus Titan oder einer Titan-Legierung in den Bereichen medizinische Geräte, Wearables, Luft- und Raumfahrt, Rennsport.
